# EUROPEAN PATENT SPECIFICATION

(11) **EP 3 365 059 B1**
(45) Date of publication and mention of the grant of the patent: **05.06.2019**
(21) Application number: 16790292.3
(22) Date of filing: 18.10.2016
(51) Int. Cl.: A61N 5/06, H05B 33/08

(54) **PHOTOTHERAPY SYSTEM**
LICHTTHERAPIESYSTEM
SYSTÈME DE PHOTOTHÉRAPIE

(30) Priority: 20.10.2015 EP 15190514
(43) Date of publication of application: 29.08.2018
(73) Proprietor: Koninklijke Philips N.V., 5656 AE Eindhoven (NL)
(72) Inventor: GROB, Timon Rutger, 5656 AE Eindhoven (NL); VAN DEN ENDE, Daan Anton, 5656 AE Eindhoven (NL)
(74) Representative: Kapoor, Pavan Puneet
(86) International application number: PCT/EP2016/074914
(87) International publication number: WO 2017/067895

(56) References cited:
- WO-A1-2007/057822
- WO-A1-2011/153599
- WO-A1-2012/048891
- WO-A1-2014/024092
- WO-A1-2015/075610
- US-A1- 2006 028 156
- US-A1- 2010 182 294
- US-A1- 2015 102 732

## Description

### FIELD OF THE INVENTION

The present invention relates to a phototherapy system and a program of operating such a phototherapy system for providing phototherapy to a subject, in particular an infant.

### BACKGROUND OF THE INVENTION

Jaundice is a condition characterized by yellowing of the skin and eyes that is caused by an excess of bilirubin in the blood. Bilirubin is a normal waste product resulting from the breakdown of red blood cells and is removed from the body by the liver. Prior to birth, bilirubin in an infant is processed by the mother's liver and excreted. After birth, an infant must eliminate bilirubin without the mother's help. It may take the infant's system several days to begin eliminating bilirubin from the blood faster than it is produced. Bilirubin levels normally peak in full-term infants 4 to 5 days after birth and may peak later in premature infants. If the infant's serum bilirubin levels continue to rise the infant is at risk for kernicterus (the deposit of bilirubin in the brain at toxic levels which can cause permanent neurologic impairment). Over half of all babies (50-60%) is affected by some level of jaundice in the first weeks of life.

For newborns diagnosed with hyperbilirubinemia, non-invasive phototherapy is considered to be the safest and most commonly used treatment. The American Academy of Pediatrics provides the following guidance when selecting a phototherapy device to treat hyperbilirubinemia in newborns:
1) emission of light in the blue-to-green spectrum (≈ 460-490 nm);
2) irradiance of at least 30 µW/cm²/nm (confirmed with an appropriate irradiance meter calibrated over the appropriate wavelength range);
3) illumination of maximal body surface; and
4) demonstration of a decrease in total bilirubin concentrations during the first 4 to 6 hours of exposure.

Generic ideas exist for closed loop control focusing on energy reduction. For instance, WO 2014/037867 A1 uses a conventional light source in combination with an additional sensor to register the presence and location of the baby and to adjust illumination areas accordingly. Recently, more and more products based on LED illumination are entering the jaundice treatment market. These are mostly based on arrays of LEDs which illuminate an area much larger than the size of the baby. A phototherapy system using LEDs as light sources is, for instance, described in WO 2014/024092 A1. The disclosed phototherapy system uses additional sensors to determine the position and/or posture of an infant to provide effective phototherapy through a set of LEDs, to avoid electromagnetic radiation from the light sources directly impinging on the eyes of the infant, and to reduce or limit the level of electromagnetic radiation leaked into the environment.

### SUMMARY OF THE INVENTION

It is an object of the present invention to provide a phototherapy system and a program of operating such a phototherapy system, which is further improved concerning the manufacturing costs and the possible applications.

In a first aspect of the present invention a phototherapy system is presented comprising
- an LED carrier for being arranged on or under a subject,
- a set of LEDs, wherein at least a first subset of LEDs of said set is arranged in or at the LED carrier,
- a control unit for controlling one or more LEDs of said first subset to switch between an emission mode, in which an LED emits electromagnetic radiation in the direction of the subject, and a detection mode, in which an LED generates a detection signal in dependence on the electromagnetic radiation incident on the LED,
- a position determination unit for determining the position of a subject with respect to the LED carrier based on the detection signals of one or more LEDs of said first subset in the detection mode, and
- a vital signs determination unit for determining one or more vital signs of the subject from one or more detection signals generated by one or more LEDs of said first subset,
wherein the control unit is configured to control one or more LEDs to switch into the emission mode based on the determined position of the subject such that their emitted electromagnetic radiation provides phototherapy for the subject.

In a further aspect of the present invention a program is presented causing a phototherapy system to carry out, when executed, the following steps:
- controlling one or more LEDs of said first subset to switch between an emission mode, in which an LED emits electromagnetic radiation in the direction of the subject, and a detection mode, in which an LED generates a detection signal in dependence on the electromagnetic radiation incident on the LED,
- determining the position of a subject with respect to the LED carrier based on the detection signals of one or more LEDs of said first subset in the detection mode,
- determining one or more vital signs of the subject from one or more detection signals generated by one or more LEDs of said first subset, and
- controlling one or more LEDs to switch into the emission mode based on the determined position of the subject such that their emitted electromagnetic radiation provides phototherapy for the subject.

Preferred embodiments of the invention are defined in the dependent claims. It shall be understood that the claimed program has similar and/or identical preferred embodiments as the claimed system, in particular as defined in the dependent claims and as disclosed herein.

The present invention is based on the idea that the use of an LED array (or a subset thereof) does not only enable easy on/off switching of parts of the illuminated area, thereby preventing illumination in areas where it is not needed, but can also be used to measure incident electromagnetic radiation to detect whether and where an infant is present, thereby eliminating the need for additional sensors for position detection, as used in known phototherapy systems. For this purpose LEDs are switched between two different modes, namely the radiation emission mode (in which LEDs are conventionally used) and the detection mode, in which an LED is used like a photo detector. The detection mode is possible since a voltage will develop over a resistor if the (non-biased) LED is illuminated with radiation (e.g. light), wherein the voltage increases with increasing intensity of the radiation. Hereby, the incident radiation should contain the wavelength that is equal or smaller than the output wavelength of the LED.

Further, the present invention also facilitates prevention of illumination in areas where it is not needed, resulting in a decrease of power usage and extended battery operation (which is e.g. useful in case of using a portable phototherapy module including the LEDs and including a battery as power supply for the LEDs). Due to the decrease in optical output power it might become feasible to increase the total intensity of, for instance, a phototherapy blanket (including the LEDs) for jaundice treatment of neonates to a value above 30 µW/cm²/nm.

According to the present invention, LEDs may thus be selectively used as light source or as sensor, i.e. no dedicated sensors are needed. This leads to an increase in battery operation time and offers the possibility to increase optical output power due to a decrease in thermal load.

According to an embodiment said control unit is configured to control an LED to switch into the emission mode, if it is configured and arranged such that a substantial amount, e.g. more than 10% or 25% or 50% (depending on the particular arrangement and implementation), of electromagnetic radiation emitted by said LED hits the subject. Hence, generally only LEDs which can efficiently provide phototherapy are switched into the emission mode, whereas LEDs from which a large part of the emitted electromagnetic radiation leaks (and does not hit the subject) are not switched into the emission mode.

Preferably, said position determination unit is configured to determine if a detection signal is above or below a predetermined threshold. The threshold may be obtained through simulation or earlier measurements. It may depend on the kind of LED that is particularly used. The use of a threshold represents a simple way to detect if the LED is covered by a part of the subject or not. Hence, in another embodiment said position determination unit may be configured to determine that an LED is underneath the subject if the detection signal generated by said LED in detection mode is below said predetermined threshold. Further, said control unit may be configured to control an LED to switch into the emission mode only if the detection signal generated by said LED in detection mode is below said predetermined threshold. Still further, said control unit may be configured to control an LED to switch off or to switch into the detection mode if the detection signal generated by said LED in detection mode is above said predetermined threshold.

According to another embodiment said set of LEDs is subdivided into groups of LEDs and said control unit is configured to commonly control the LED of a group to switch into the emission mode, if a detection signal generated by one or more LEDs of said group in detection mode is below said predetermined threshold. Thus, not each single LED needs to be switched between modes, but only selected ones, and less detection signals need to be evaluated. The number of such LEDs and the size of the groups may e.g. depend on the total number of LEDs, their spacing and the desired accuracy of the position determination.

In another embodiment said control unit is configured to control LEDs of said first subset, which are not switched into the emission mode, to switch into the detection mode, wherein said position determination unit is configured to continuously or regularly evaluate detection signals generated by LEDs in the detection mode to determine if the position of the subject with respect to the LED carrier has changed, and wherein said control unit is configured to change the control of the LEDs if it is determined that the position of the subject with respect to the LED carrier has changed. Thus, a continuous monitoring of the subject movement and update of the control of the LEDs is possible.

There are different mechanical configurations of the phototherapy system possible. According to one construction all LEDs of said set are arranged in or at the LED carrier. The LED carrier may e.g. be a cuboid, whose upper layer may e.g. be a transparent or translucent plate, e.g. made of plastic or glass. All the LEDs may be arranged inside the LED carrier so as to emit electromagnetic radiation through this transparent or translucent plate. The LED carrier may also be a supporting body, such as a mattress, for supporting the subject on the top surface thereof. In other embodiments the LED carrier may be a bed sheet or blanket for covering or contacting the subject from the top, side or below, into which the LEDs may be integrated.

According to another construction one or more LEDs of a second subset of said set are arranged to be placed at two or more different sides of the subject, e.g. above, below and/or at a side of the subject, such that electromagnetic radiation emitted by the LEDs of said second subset hits the subject from above, below and/or from one or more sides. Hence, phototherapy may not only be provided from a single direction (i.e. from below the subject), but also from one or more additional directions. Such subsets of LEDs may e.g. be arranged within an incubator, e.g. at the cover plate or below the cover of the incubator. Further, the LED carrier may be configured to be placed at different sides of the subject, such as a blanket.

In such a configuration said control unit may be further configured to control an LED of said second subset to switch into the emission mode only if the detection signal generated by an LED of the first subset at the same longitudinal position with respect to the subject is below said predetermined threshold. Hence, based on detection signals of LEDs of the first subset also LEDs of the second subset are controlled, preferably in such a way that it is estimated which LEDs of the second subset can efficiently provide phototherapy based on the detection signals of the first subset. One option is to control LEDs at a certain longitudinal position (i.e. a position in the direction of the length of the subject) based on the detection signal of an LED of the first subset at the same longitudinal position (or close to it in a certain range (e.g. +/- 1cm)).

Further, in such a configuration said control unit may be further configured to control an LED of said second subset to switch off if the detection signal generated by an LED of the first subset at the same longitudinal position with respect to the subject is above said predetermined threshold. This further reduces leakage of radiation and saves energy.

The phototherapy system according to the above described aspect of the present invention further comprises a vital signs determination unit for determining one or more vital signs of the subject from one or more detection signals generated by one or more LEDs of said first subset. Conventionally, in known phototherapy equipment, additional equipment or sensors are used for measurements of vital signs, such as cameras or temperature sensors. These additional sensors are not needed when selected LEDs are used for the sensing functionality. For instance, for acquiring photoplethysmography (PPG) signals for deriving an SpO2 signal of the subject, three LEDs may be used, where a first LEDs emits red light onto the skin of the subject, a second LED emits infrared light onto the skin of the subject and a third LED detects (in detection mode) light reflected from the irradiated skin to obtain a PPG signal from which the SpO2 information can be derived in a known manner (as e.g. conventionally done in an SpO2 sensor often used as a finger clip). In another embodiment one or more LEDs may be used in detection mode for temperature measurement, breathing rate measurement or heart rate measurement of the subject's body temperature, breathing rate or heart rate. Thus, the LEDs may be used for different functions, which saves costs and space since no additional means for vital signs detection need to be provided.

In still another embodiment said position determination unit is configured to measure an open circuit voltage of an LED of said first subset in detection mode as detection signal, which represents a simple way of obtaining the detection signal. Another method of obtaining the detection signal is by reverse biasing the LED (i.e. with the cathode driven positive with respect to the anode) and measuring the current change when the LED is illuminated. The advantage of reverse biased measurement is the increased acquisition speed of such a measurement.

The control unit may further be configured to switch only LEDs of said first subset between said emission mode and said detection mode. Hence, if there are further LEDs provided, e.g. forming a second subset that is not part of the LED carrier, these further LEDs are not switched between said emission mode and said detection mode, but are only used in emission mode (or are switched off). This reduces the complexity of the control unit.

Further, the control unit may be configured to switch at least one or more LEDs of said first subset into the emission mode based on the determined position of the subject. Additional LEDs, e.g. of a second subset, may be used in emission mode as well. In a simple embodiment, however, only the first subset of LEDs is available (i.e. all LEDs are part of the LED carrier, which are used in detection mode and in emission mode. This provides a space- and cost-saving embodiment of the phototherapy system.

A further phototherapy system comprises:
- an LED carrier for being arranged on or under a subject,
- a set of LEDs, wherein at least a first subset of LEDs of said set is arranged in or at the LED carrier,
- a control unit for controlling one or more LEDs of said first subset to switch between an emission mode, in which an LED emits electromagnetic radiation in the direction of the subject, and a detection mode, in which an LED generates a detection signal in dependence on the electromagnetic radiation incident on the LED, and
- a position determination unit for determining the position of a subject with respect to the LED carrier based on the detection signals of one or more LEDs of said first subset in the detection mode,
wherein the control unit is configured to control one or more LEDs to switch into the emission mode based on the determined position of the subject such that their emitted electromagnetic radiation provides phototherapy for the subject and to switch only LEDs of said first subset between said emission mode and said detection mode.

A further aspect program causes a phototherapy system to carry out, when executed, the following steps:
- controlling one or more LEDs of said first subset to switch between an emission mode, in which an LED emits electromagnetic radiation in the direction of the subject, and a detection mode, in which an LED generates a detection signal in dependence on the electromagnetic radiation incident on the LED,
- determining the position of a subject with respect to the LED carrier based on the detection signals of one or more LEDs of said first subset in the detection mode, and
- controlling one or more LEDs to switch into the emission mode based on the determined position of the subject such that their emitted electromagnetic radiation provides phototherapy for the subject and to switch only LEDs of said first subset between said emission mode and said detection mode.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a phototherapy system according to the present invention,
Fig. 2 shows a diagram of the typical photodiode current-voltage characteristics of an LED,
Fig. 3 shows a schematic diagram of a second embodiment of a phototherapy system according to the present invention,
Fig. 4 shows a schematic diagram of the third embodiment of the phototherapy system according to the present invention, and
Fig. 5 shows a flow chart of an embodiment of the method according to the present invention.

### DETAILED DESCRIPTION OF THE INVENTION

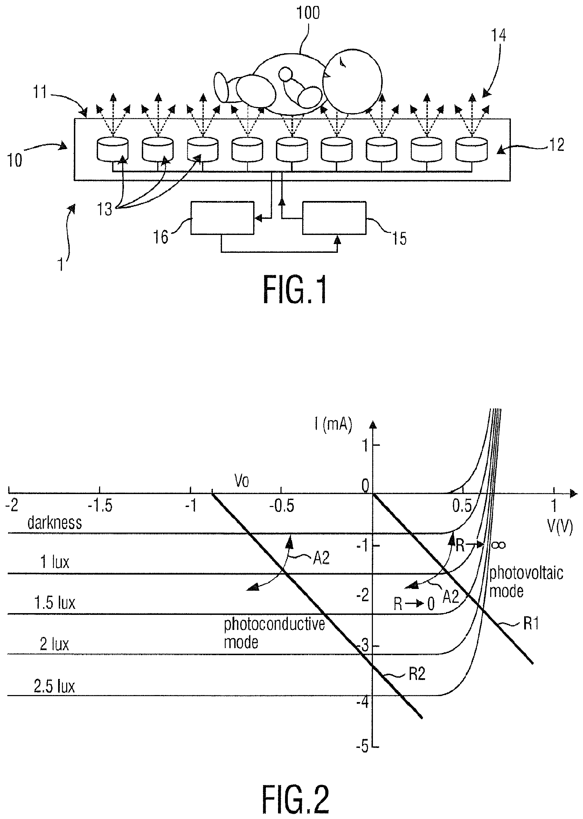

Fig. 1 shows a schematic diagram of a first embodiment of a phototherapy system 1 according to the present invention. The phototherapy system 1 comprises an LED carrier 10, e.g. a bed, mattress, patient table, blanket, etc. In the exemplary embodiment shown in Fig, 1 the LED carrier 10 is configured as a mattress for supporting a subject 100, e.g. an infant (e.g. a neonate), on a top surface 11 thereof. A set 12 of LEDs 13 is arranged in or at the LED carrier 10, wherein in this embodiment all LEDs 13 are arranged inside the LED carrier such that they emit electromagnetic radiation 14 through the top surface 11 (which is transparent for the emitted electromagnetic radiation 14) such that at least part thereof directly impinges on subject 100 and thus provide phototherapy to the subject.

Phototherapy is particularly used to treat jaundice (or hyperbilirubinemia) by reducing the level of bilirubin. Effective and/or appropriate levels of phototherapy may be based on the subject's age, size, weight, and/or other physiological, environmental, and/or subject-specific parameters. Phototherapy preferably uses electromagnetic radiation having a peak wavelength between, e.g., 460 nm and 500 nm, an emission spectrum ranging from, e.g., 400 nm to 520 nm, and preferably using a narrow bandwidth delivered at an irradiance of, e.g., 30-35 µW/cm2/nm to, e.g., up to 80% of a subject's body surface area (BSA). Phototherapy may also be applied to treat other problems, such as acne.

The phototherapy system 1 further comprises a control unit 15 for controlling one or more LEDs 13 of said set 12 to switch between an emission mode, in which an LED emits electromagnetic radiation in the direction of the subject 100, and a detection mode, in which an LED generates a detection signal in dependence on the electromagnetic radiation incident on the LED. Hence, single LEDs, groups of LEDs or all LEDs can be operated in those two different modes, in which they either function as a conventional radiation emitting element or as a radiation detection element (similar to a photo detector or photodiode) as will be explained below in more detail.

The phototherapy system 1 further comprises a position determination unit 16 for determining the position of a subject 100 on the top surface 11 of the LED carrier 10 based on the detection signals of one or more LEDs 13 (e.g. of all LEDs) in the detection mode. Hereby, the effect is evaluated that an LED in the detection mode provides an output signal, whose intensity is dependent on the intensity of the incident electromagnetic radiation, particularly in the spectral range, in which the LED emits electromagnetic radiation in the emission mode. Hence, if an LED outputs a detection signal with a small (or zero) amplitude indicating a small intensity of incident radiation, it is interpreted such that this LED is covered by the subject's body and no ambient radiation (or radiation from a dedicated illumination source (not shown in Fig. 1)) is incident on the LED. On the other hand, if an LED outputs a detection signal with comparably large amplitude indicating a larger intensity of incident radiation, it is interpreted such that this LED is not covered by the subject's body and ambient radiation (or radiation from a dedicated illumination source) is incident on the LED. The detection signals thus allow determining the position of the subject 100.

The control unit 15 and the position determination unit 16 are preferably implemented by a common element or separate elements, e.g. a common or separate processor or programmable elements, which are programmed accordingly.

The control unit 15 is hence configured to control one or more LEDs 13 to switch into the emission mode based on the determined position of the subject 100 such that their emitted electromagnetic radiation provides phototherapy for the subject 100.

Hereby, in one embodiment, all LEDs 13 are used in both modes, so that in this case all LEDs which have shown a detection signal with comparably small (or zero) amplitude (indicating that they are covered by the subject 100; in Fig. 1 e.g. the fourth to seventh LEDs, counted from the left side) will be switched into the emission mode to provide phototherapy. All other LEDs 13 (in Fig. 1 e.g. the three LEDs on the left side and the two LEDs on the right side) will either be switched off or kept in the detection mode (which may be the same in case the detection mode is effected by disconnecting the LED from its power supply to switch it into detection mode) to continuously monitor if any position changes of the subject's position happen.

Preferably, the control unit 15 is configured to control an LED 13 to switch into the emission mode, if it is configured and arranged such that a substantial amount, e.g. more than 10, 25, or 50% (or any other percentage determined in the particular application, of electromagnetic radiation emitted by said LED 13 hits the subject 100. This could be directly measured with the position determination unit 16, which may be configured to determine if a detection signal is above or below a predetermined threshold. The threshold is generally predetermined, e.g. through calibration measurements, trials or simulation.

For instance, the position determination unit 16 may be configured to determine that an LED 13 is underneath the subject 100 if the detection signal generated by said LED 13 in detection mode is below said predetermined threshold. The control unit 15 is then preferably configured to control an LED 13 to switch into the emission mode only if the detection signal generated by said LED 13 in detection mode is below said predetermined threshold and/or to control an LED 13 to switch off or to switch into the detection mode if the detection signal generated by said LED 13 in detection mode is above said predetermined threshold.

In another embodiment the control unit 15 is configured to control one or more LEDs 13, which are not switched into the emission mode, to switch into the detection mode and the position determination unit 16 is configured to continuously or regularly evaluate detection signals generated by LEDs 13 in the detection mode to determine if the position of the subject 100 with respect to the LED carrier 10 (in this embodiment on the top surface 11 of the mattress) has changed. If the position of the subject 100 with respect to the LED carrier 10 has indeed changed and if this has been detected by the evaluation of the detection signals, the control unit 15 changes the control of the LEDs 13, i.e. changes the modes for one or more LEDs to make sure that only LEDs, whose radiation substantially impinges on the subject 100 are in the emission mode.

In another embodiment only some of the LEDs 13 (i.e. a subset thereof, e.g. each second LED) is used in both modes, i.e. the other LEDs are either switched on (to emit radiation) or off (not to emit radiation). The latter LEDs are then controlled according to the detection signal(s) of one or more of the neighboring LEDs, which are used in both modes. Hence, in general, the LEDs may be grouped into groups, where only one or more LEDs of the group is used in both modes and the detection signal of said LED(s) controls the state of the other or all LEDs of the group. In other words, the control unit 15 commonly controls the LEDs of a group to switch into the emission mode, if a detection signal generated by one or more LEDs of said group in detection mode is below said predetermined threshold.

The open circuit voltage can be measured across an LED in detection mode as a function of illumination of the LED. The LED then behaves like a photodiode when illuminated and is sensitive to incident radiation (in particular light) with a wavelength equal or smaller than the output wavelength of the LED. When the LED is illuminated with a radiation intensity above 0, a voltage will develop over a resistor (e.g. included in the control unit 15 or provided as separate element), for instance a voltmeter. This is illustrated in Fig. 2 showing a diagram of the typical current-voltage characteristics of an LED. As can be seen there, an LED in detection mode can be operated both in photovoltaic mode as well as in photoconductive mode, when the LED is negatively biased. As shown, when no voltage is applied to the LED the output voltage (which is one embodiment of the detection signal) increases with increasing intensity (in this case lux) of the incident radiation. In both the emission mode and in the detection mode the LEDs can be pulsed. In Fig. 2 the lines R1 and R2 indicate the voltage-current relation for a given resistance and the double arrows A1 and A2 indicate how the slope of these lines changes when the resistance changes.

There are generally two ways to measure: photovoltaic mode, where the LED generates a voltage as function of the incident light, and photoconductive mode where the change in current flowing through the LED is measured as a function of incident light. The photoconductive mode is reverse biased and the photovoltaic mode is typically unbiased, but can be maintained up to a small positive bias (i.e. under the threshold voltage for the LED as also shown in Fig. 2). The photoconductive and photovoltaic modes both fall under the 'detection mode' definition, while the region above the threshold for light emission of the LED (i.e. when the current becomes positive, for instance at about 0.6V to 0.7V in the graph in Fig. 2). Any voltage that is applied above 0.7 V will cause the LED to emit light (i.e. the LED is set to emission mode).

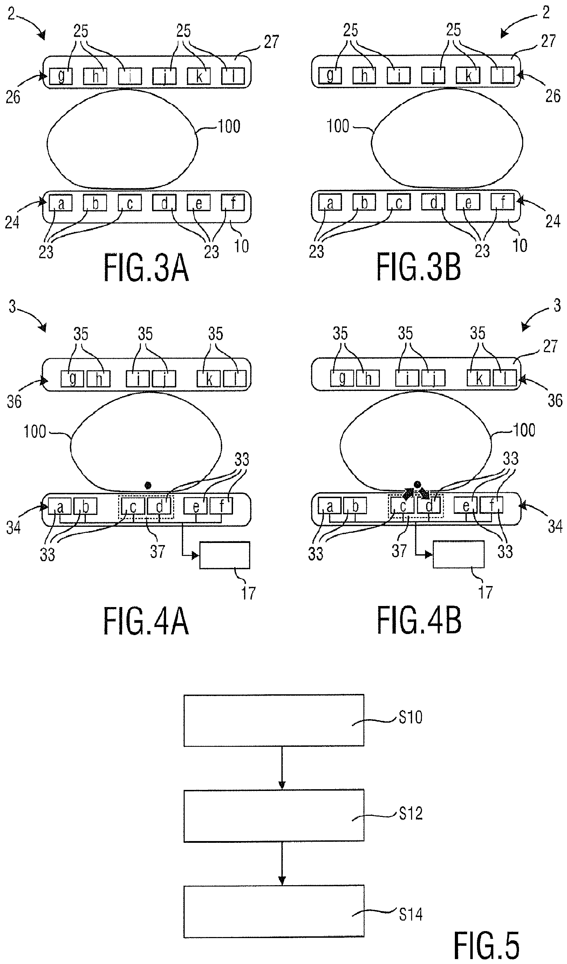

Fig. 3 shows a schematic diagram of a second embodiment of a phototherapy system 2 according to the present invention, wherein the subject 100 is at a first position in Fig. 3A and at a second position in Fig. 3B. In this embodiment the total set 22 of LEDs comprises a first subset 23 of LEDs 24 arranged in or at the LED carrier 10 and a second subset 25 of LEDs 26 arranged in or at one or more other LED carriers 27 (such as a side wall, frame, ceiling, etc. of e.g. an incubator or other assembly (e.g. a baby crib) or integrated in a flexible blanket arranged around the subject 100; alternatively the LED carrier 10 and the one or more other carriers 27 may also be a common LED carrier). The position determination unit 15 and the control unit 16 are not shown in this embodiment.

The LEDs 24 of the first subset 23 are generally configured in the same way as the LEDs 13 of the first embodiment of the phototherapy system 1. If the LEDs 25 of the second subset 26 are not in contact with the subject 100, they are preferably configured to emit radiation only or to be switched off, but they are generally not used to be switched between the emission mode and the detection mode like the LEDs 23 since other stray light may reach these LEDs even if an LED is directly above the subject making position detection inaccurate if not impossible.

The control unit 15 controls one or more LED 25 of said second subset 26 to switch into the emission mode only if the detection signal generated by an LED 23 of the first subset 24 at the same longitudinal position with respect to the subject 100 is below the above mentioned predetermined threshold, i.e. if it indicates that no or not much radiation is received because it is covered by the subject. For instance, in the position of the subject 100 shown in Fig. 3A the LED b is covered by the subject 100 and will output no or only a small detection signal. The control unit 15 will switch the LED b and the LED h (which is at the same longitudinal position as the LED b) into the emission mode. In contrast LED f is not covered by the subject 100 and will thus output a larger detection signal so that the control unit 15 will not switch the LED 1 (which is at the same longitudinal position as the LED f, which is also not switched on for the rest of the treatment) into the emission mode (but will e.g. be switched off). In the position of the subject 100 shown in Fig. 3B, in contrast, LEDs g and a will be switched off and LEDs k and e will be used in emission mode.

The LEDs 23 of the first subset 24 are generally used and controlled in the same manner as the LEDs 13 of the set 12 of the first embodiment.

Hence, according to the present invention movement sensing can be achieved using the LEDs. Generally, when the subject 100 changes position, the output of the LEDs in detection mode changes. This can trigger a change of the control or even an alert. To determine the position of the infant more accurately the response of individually addressed LEDs can be determined. The individual responses (detection signals) can be mapped to determine where the subject is positioned and even how it is oriented.

The position sensing could also be done in the same plane by measuring the response of the adjacent LED. This shall be explained by referring to the subset 26 of LEDs 25 shown in Fig. 3A. When the LED g is in emission mode and the other LEDs are in detection mode the adjacent LED h measures light. When the LED h is in emission mode and the other LEDs are in detection mode the adjacent LED g measures light but the LED i does not because it is blocked by the subject's body. When the LED i is in emission mode and the other LEDs are in detection mode the adjacent LEDs h and j do not measure light because the source LED i is blocked by the subject's body. When the LED j is in emission mode and the other LEDs are in detection mode the adjacent LED k measures light but the LED i does not. When the LED k is in emission mode and the other LEDs are in detection mode the adjacent LEDs j and 1 measure light. Hence the measurement confirms that LED i is blocked.

Fig. 4 shows third embodiment of the phototherapy system 3 according to the present invention, which may additionally be applied for SpO2 measurements. Like in Fig. 3, the subject 100 is at a first position in Fig. 4A and at a second position in Fig. 4B. This embodiment is partly similar in that a first set 34 of LEDs 33 is arranged in the LED carrier 10 and a second set 36 of LEDs 35 is arranged in or at another LED carrier 27.

When the subject is not (or not much) moving (for instance when it is asleep), vital signs (such as heart rate, respiration rate, SpO2, etc.) can be measured using a part of the set of LEDs, which are switchable between detection mode and emission mode as conventional LED and photo detector, as e.g. used in a conventional pulse oximeter, for acquiring photoplethysmography (PPG) signals. For the pulse oximeter to work optimally for SpO2, the LEDs emit red and infrared (IR) light. This can e.g. be achieved by positioning pairs of a red and infrared LEDs (one for emission and one for detection) between the other (e.g. blue) LEDs used for performing phototherapy. In an alternative embodiment, LEDs or LED units that can be switched between different colors, may be used. For instance, a configurable RGB-IR LED chip, which contains multiple LEDs on one die, can be used for this purpose.

In Fig. 4 the LEDs 33 of the first subset 34 are grouped into LED units (e.g. LEDs unit 37 indicated as example) of two LEDs (e.g. LEDs c and e in LED unit 37) each, as shown in Fig. 4 (or one configurable RGB-IF LED chip). Each LED unit 37 can be switched between emission mode (to emit radiation, e.g. blue light, for phototherapy), detection mode and vital signs mode for acquiring a vital sign signal or information from which a vital sign of the subject can be derived. If close enough to the subject 100, the LEDs 35 of the second subset 36 may also be used for acquisition of vital sign signals or information from which a vital sign of the subject can be derived.

When not in vital signs mode the LEDs 33 (including the LED units 37) and 35, can emit radiation for phototherapy in emission mode and some (or all) of the LEDs 33 are in detection mode for detecting the subject's position (illustrated in Fig. 4A). For SpO2 measurements the LED unit 37 can both emit and receive red and IR wavelengths, wherein the difference in response can be used to sense SpO2, as conventionally known from pulse oximeters. The LED unit 37 comprising the LEDs c and d, where the subject 100 is positioned, is then switched into the vital signs mode to record the signals for reflective SpO2 measurement. For this purpose, e.g., the LED c emits red and IR light and the LED d receives red and IR light reflected from the subject's skin. The received signals are then processed by a vital signs determination unit 17 (which may be implemented by the separate processor or the same processor as used for the control unit 15 and/or the position determination unit 16) for determining one or more vital signs of the subject from one or more detection signals generated by the LED d.

In an embodiment, the red and infrared LEDs are not sensitive to blue light, for instance by providing a filter that blocks blue light, so that the SpO2 measurement is not influenced by the phototherapy treatment of the other (blue) LEDs, and the SpO2 measurement can thus be performed without interrupting the phototherapy.

The present invention can also be used for vital signs measurement of other vital signs. For instance, respiratory measurements may be conducted by measuring the photo induced voltage as a function of the breathing induced movements. In this case the voltage induced by the LEDs in detection mode is dependent on the (dynamic) shape of the subject's body. When the body moves, the total registered output voltage of all LEDs in detection mode (e.g. all LEDs arranged in or at the LED carrier) changes also. The periodicity of these changes can then be interpreted as the respiration rate.

Alternatively, a single LED can be used for measurements. This requires individual addressing of the LEDs, but increases the accuracy of the measurement. Multiple LEDs can be addressed sequentially in a sweep to determine the position of the most sensitive LED(s). This LED is subsequently used for monitoring the respiratory movement. If the subject moves, this is registered as a large change in the (DC) signal of the LED, which triggers a new sweep to determine the most sensitive LED for the new position.

LEDs are generally temperature dependent. If the temperature dependence of measurement LEDs is known (e.g. calibrated), these LEDs can be used as temperature sensors, enabling monitoring of the temperature of the subject during operation. In this case the LED junction temperature is calculated from the current-voltage characteristics (and changes therein). If the position of the subject is known (using the method disclosed herein), it is known whether the LED is in contact with the baby or not. This influences the temperature of the LED for a given driving current, because it influences the ambient temperature in the local vicinity of the LED. A comparison can be made between an LED which is in contact with the subject (subject temperature) and an LED which is not in contact with the subject (room temperature).

Fig. 5 shows a flow chart of an embodiment of the method of operating a phototherapy system according to the present invention. In a first step S10 one or more LEDs of said first subset of the phototherapy device are set to switch between an emission mode, in which an LED emits electromagnetic radiation in the direction of the subject, and a detection mode, in which an LED generates a detection signal in dependence on the electromagnetic radiation incident on the LED. In a second step S12 the position of a subject with respect to the LED carrier is determined based on the detection signals of one or more LEDs of said first subset in the detection mode. In a third step S14 one or more LEDs are controlled to switch into the emission mode based on the determined position of the subject such that their emitted electromagnetic radiation provides phototherapy for the subject.

In summary, according to the present invention a phototherapy system and a corresponding control method are presented using only LEDs as sensing system for position detection and phototherapy treatment (and, optionally, vital signs measurements). Such a combination of multiple measurements in one type of sensor is useful and interesting because of its simplicity.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A phototherapy system comprising:
- an LED carrier (10) for being arranged on or under a subject (100),
- a set (12; 24, 26; 36) of LEDs (13; 23, 25; 33, 35), wherein at least a first subset (12; 24; 34) of LEDs (13; 23; 33) of said set is arranged in or at the LED carrier (10),
- a control unit (15) for controlling one or more LEDs (13; 23; 33) of said first subset (12; 24; 34) to switch between an emission mode, in which an LED emits electromagnetic radiation in the direction of the subject, and a detection mode, in which an LED generates a detection signal in dependence on the electromagnetic radiation incident on the LED,
- a position determination unit (16) for determining the position of a subject with respect to the LED carrier based on the detection signals of one or more LEDs (13; 23; 33) of said first subset (12; 24; 34) in the detection mode, and
- a vital signs determination unit (17) for determining one or more vital signs of the subject from one or more detection signals generated by one or more LEDs of said first subset,
wherein the control unit (15) is configured to control one or more LEDs (13; 23, 25; 33, 35) to switch into the emission mode based on the determined position of the subject such that their emitted electromagnetic radiation provides phototherapy for the subject.

2. The phototherapy system as claimed in claim 1,
wherein said control unit (15) is configured to control an LED to switch into the emission mode, if it is configured and arranged such that a substantial amount of electromagnetic radiation emitted by said LED hits the subject.

3. The phototherapy system as claimed in claim 1,
wherein said position determination unit (16) is configured to determine if a detection signal is above or below a predetermined threshold.

4. The phototherapy system as claimed in claim 3,
wherein said position determination unit (16) is configured to determine that an LED is underneath the subject if the detection signal generated by said LED in detection mode is below said predetermined threshold.

5. The phototherapy system as claimed in claim 4,
wherein said control unit (15) is configured to control an LED to switch into the emission mode only if the detection signal generated by said LED in detection mode is below said predetermined threshold.

6. The phototherapy system as claimed in claim 3,
wherein said control unit (15) is configured to control an LED to switch off or to switch into the detection mode if the detection signal generated by said LED in detection mode is above said predetermined threshold.

7. The phototherapy system as claimed in claim 3,
wherein said set of LEDs (13; 23, 25; 33, 35) is subdivided into groups of LEDs and wherein said control unit (15) is configured to commonly control the LED of a group to switch into the emission mode, if a detection signal generated by one or more LEDs of said group in detection mode is below said predetermined threshold.

8. The phototherapy system as claimed in claim 1,
wherein said control unit (15) is configured to control LEDs of said first subset, which are not switched into the emission mode, to switch into the detection mode,
wherein said position determination unit (16) is configured to continuously or regularly evaluate detection signals generated by LEDs in the detection mode to determine if the position of the subject with respect to the LED carrier has changed, and
wherein said control unit (15) is configured to change the control of the LEDs if it is determined that the position of the subject with respect to the LED carrier has changed.

9. The phototherapy system as claimed in claim 1,
wherein all LEDs (13) of said set (12) are arranged in or at the LED carrier (10).

10. The phototherapy system as claimed in claim 1,
wherein one or more LEDs (25; 35) of a second subset (26; 36) of said set are arranged to be placed at two or more different sides of the subject such that electromagnetic radiation emitted by the LEDs (25; 35) of said second subset hits the subject from above, below and/or from one or more sides.

11. The phototherapy system as claimed in claim 10,
wherein said control unit (15) is configured to control an LED (25; 35) of said second subset (26; 36) to switch into the emission mode only if the detection signal generated by an LED (23; 33) of the first subset (24; 34) at the same longitudinal position with respect to the subject is below said predetermined threshold and/or to switch off if the detection signal generated by an LED (23; 33) of the first subset (24; 34) at the same longitudinal position with respect to the subject is above said predetermined threshold.

12. The phototherapy system as claimed in claim 1,
wherein said control unit (15) is configured to switch only LEDs (13; 23; 33) of said first subset (12; 24; 34) between said emission mode and said detection mode.

13. The phototherapy system as claimed in claim 1,
wherein said control unit (15) is configured to switch at least one or more LEDs (13; 23; 33) of said first subset (12; 24; 34) into the emission mode based on the determined position of the subject.

14. The phototherapy system as claimed in claim 1,
wherein said position determination unit (16) is configured to measure an open circuit voltage of an LED of said first subset in detection mode as detection signal.

15. A program causing a phototherapy system comprising an LED carrier (10) for being arranged on or under a subject (100) and a set (13; 23, 25; 33, 35) of LEDs (12; 24, 26; 36), wherein at least a first subset (12; 24; 34) of LEDs (13; 23; 33) of said set is arranged in or at the LED carrier (10), to carry out, when executed, the following steps:
- controlling one or more LEDs (13; 23; 33) of said first subset (12; 24; 34) to switch between an emission mode, in which an LED emits electromagnetic radiation in the direction of the subject, and a detection mode, in which an LED generates a detection signal in dependence on the electromagnetic radiation incident on the LED,
- determining the position of a subject with respect to the LED carrier based on the detection signals of one or more LEDs (13; 23; 33) of said first subset (12; 24; 34) in the detection mode,
- determining one or more vital signs of the subject from one or more detection signals generated by one or more LEDs of said first subset, and
- controlling one or more LEDs (13; 23, 25; 33, 35) to switch into the emission mode based on the determined position of the subject such that their emitted electromagnetic radiation provides phototherapy for the subject.

## Patentansprüche

1. Lichttherapiesystem umfassend:
- einen LED-Träger (10) zur Anordnung auf oder unter einem Subjekt (100),
- eine Gruppe (12; 24, 26; 36) von LEDs (13; 23, 25; 33, 35), wobei mindestens eine erste Teilgruppe (12; 24; 34) von LEDs (13; 23; 33) der genannten Gruppe in oder bei dem LED-Träger (10) angeordnet ist,
- eine Steuereinheit (15) zum Steuern einer oder mehrerer LEDs (13; 23; 33) der genannten ersten Teilgruppe (12; 24; 34), um zwischen einem Emissionsmodus, in dem eine LED elektromagnetische Strahlung in Richtung des Subjekts emittiert, und einem Detektionsmodus, in dem eine LED ein Detektionssignal in Abhängigkeit von der auf die LED auftreffenden elektromagnetischen Strahlung erzeugt, umzuschalten,
- eine Positionsbestimmungseinheit (16) zum Bestimmen der Position eines Subjekts in Bezug auf den LED-Träger basierend auf den Detektionssignalen von einer oder mehreren LEDs (13; 23; 33) der genannten ersten Teilgruppe (12; 24; 34) in dem Detektionsmodus, und
- eine Vitalzeichenbestimmungseinheit (17) zum Bestimmen von einem oder mehreren Vitalzeichen des Subjekts anhand von einem oder mehreren Detektionssignalen, die durch die eine oder mehrere LEDs der genannten ersten Teilgruppe erzeugt wurden, wobei die Steuereinheit (15) konfiguriert ist, um eine oder mehrere LEDs (13; 23, 25; 33, 35) zu steuern, um basierend auf der bestimmten Position des Subjekts in den Emissionsmodus umzuschalten, sodass ihre emittierte elektromagnetische Strahlung Lichttherapie für das Subjekt bereitstellt.

2. Lichttherapiesystem nach Anspruch 1,
wobei die genannte Steuereinheit (15) konfiguriert ist, um eine LED zu steuern, um in den Emissionsmodus umzuschalten, wenn sie derartig konfiguriert und eingerichtet ist, dass eine erhebliche Menge der durch die genannte LED emittierten elektromagnetischen Strahlung auf das Subjekt auftritt.

3. Lichttherapiesystem nach Anspruch 1,
wobei die genannte Positionsbestimmungseinheit (16) konfiguriert ist, um zu bestimmen, ob ein Detektionssignal über oder unter einem vorgegebenen Schwellenwert liegt.

4. Lichttherapiesystem nach Anspruch 3,
wobei die genannte Positionsbestimmungseinheit (16) konfiguriert ist, um zu bestimmen, dass sich eine LED unterhalb des Subjekts befindet, wenn das durch die genannte LED im Detektionsmodus erzeugte Detektionssignal unter dem genannten vorgegebenen Schwellenwert liegt.

5. Lichttherapiesystem nach Anspruch 4,
wobei die genannte Steuereinheit (15) konfiguriert ist, um eine LED zu steuern, um nur dann in den Emissionsmodus umzuschalten, wenn das durch die genannte LED im Detektionsmodus erzeugte Detektionssignal unter dem genannten vorgegebenen Schwellenwert liegt.

6. Lichttherapiesystem nach Anspruch 3,
wobei die genannte Steuereinheit (15) konfiguriert ist, um eine LED zu steuern, um sich auszuschalten oder in den Detektionsmodus umzuschalten, wenn das durch die genannte LED im Detektionsmodus erzeugte Detektionssignal über dem genannten vorgegebenen Schwellenwert liegt.

7. Lichttherapiesystem nach Anspruch 3,
wobei die genannte Gruppe von LEDs (13; 23, 25; 33, 35) in Gruppen von LEDs unterteilt ist und wobei die genannte Steuereinheit (15) konfiguriert ist, um die LED einer Gruppe zu steuern, um gemeinsam in den Emissionsmodus umzuschalten, wenn ein durch eine oder mehrere LEDs der genannten Gruppe im Detektionsmodus erzeugtes Detektionssignal unterhalb dem genannten vorgegebenen Schwellenwert liegt.

8. Lichttherapiesystem nach Anspruch 1,
wobei die genannte Steuereinheit (15) konfiguriert ist, um die LEDs der genannten ersten Teilgruppe, die nicht in den Emissionsmodus umgeschaltet werden, zu steuern, um in den Detektionsmodus umzuschalten,
wobei die genannte Positionsbestimmungseinheit (16) konfiguriert ist, um durch die LEDs im Detektionsmodus erzeugte Detektionssignale kontinuierlich oder regelmäßig zu evaluieren, um zu bestimmen, ob sich die Position des Subjekts in Bezug auf den LED-Träger geändert hat, und
wobei die genannte Steuereinheit (15) konfiguriert ist, um die Steuerung der LEDs zu ändern, wenn bestimmt wird, dass sich die Position des Subjekts in Bezug auf den LED-Träger geändert hat.

9. Lichttherapiesystem nach Anspruch 1,
wobei alle LEDs (13) der genannten Gruppe (12) in oder bei dem LED-Träger (10) angeordnet sind.

10. Lichttherapiesystem nach Anspruch 1,
wobei eine oder mehrere LEDs (25; 35) einer zweiten Teilgruppe (26; 36) der genannten Gruppe dafür eingerichtet sind, auf zwei oder mehr verschiedenen Seiten des Subjekts platziert zu werden, sodass die durch die LEDs (25; 35) der genannte zweiten Teilgruppe emittierte elektromagnetische Strahlung von oben, von unten und/oder von einer oder mehreren Seiten aus auf das Subjekt auftrifft.

11. Lichttherapiesystem nach Anspruch 10,
wobei die genannte Steuereinheit (15) konfiguriert ist, um eine LED (25; 35) der genannten zweiten Teilgruppe (26; 36) zu steuern, um nur dann in den Emissionsmodus umzuschalten, wenn das durch eine LED (23; 33) der ersten Teilgruppe (24; 34) bei der gleichen longitudinalen Position in Bezug auf das Subjekt erzeugte Detektionssignal unter dem genannten vorgegebenen Schwellenwert liegt, und/oder um sich auszuschalten, wenn das durch eine LED (23; 33) der genannten ersten Teilgruppe (24; 34) bei der gleichen longitudinalen Position in Bezug auf das Subjekt erzeugte Detektionssignal über dem genannten vorgegebenen Schwellenwert liegt.

12. Lichttherapiesystem nach Anspruch 1,
wobei die genannte Steuereinheit (15) konfiguriert ist, um nur LEDs (13; 23; 33) der genannten ersten Teilgruppe (12; 24; 34) zwischen dem genannten Emissionsmodus und dem genannten Detektionsmodus umzuschalten.

13. Lichttherapiesystem nach Anspruch 1,
wobei die genannte Steuereinheit (15) konfiguriert ist, um mindestens eine oder mehrere LEDs (13; 23; 33) der genannten ersten Teilgruppe (12; 24, 34) basierend auf der bestimmten Position des Subjekts in den Emissionsmodus umzuschalten.

14. Lichttherapiesystem nach Anspruch 1,
wobei die genannte Positionsbestimmungseinheit (16) konfiguriert ist, um eine Leerlaufspannung einer LED der genannten ersten Teilgruppe im Detektionsmodus als Detektionssignal zu messen.

15. Programm, das ein Lichttherapiesystem umfassend einen LED-Träger (10) zur Anordnung auf oder unter einem Subjekt (100) und eine Gruppe (13; 23, 25; 33, 35) von LEDs (12; 24, 26; 36), wobei mindestens eine erste Teilgruppe (12; 24; 34) von LEDs (13; 23; 33) der genannten Gruppe in oder bei dem LED-Träger (10) angeordnet ist, dazu veranlasst, wenn es ausgeführt wird, die folgenden Schritte durchzuführen:
- Steuern einer oder mehrerer LEDs (13; 23; 33) der genannten ersten Teilgruppe (12; 24; 34), um zwischen einem Emissionsmodus, in dem eine LED elektromagnetische Strahlung in Richtung des Subjekts emittiert, und einem Detektionsmodus, in dem eine LED ein Detektionssignal in Abhängigkeit von der auf die LED auftreffenden elektromagnetischen Strahlung erzeugt, umzuschalten,
- Bestimmen der Position eines Subjekts in Bezug auf den LED-Träger basierend auf den Detektionssignalen von einer oder mehreren LEDs (13; 23; 33) der genannten ersten Teilgruppe (12; 24; 34) in dem Detektionsmodus,
- Bestimmen von einem oder mehreren Vitalzeichen des Subjekts anhand von einem oder mehreren Detektionssignalen, die durch die eine oder mehrere LEDs der genannten ersten Teilgruppe erzeugt wurden, und
- Steuern von einer oder mehreren LEDs (13; 23, 25; 33, 35), um basierend auf der bestimmten Position des Subjekts in den Emissionsmodus umzuschalten, sodass ihre emittierte elektromagnetische Strahlung Lichttherapie für das Subjekt bereitstellt.

## Revendications

1. Système de photothérapie comprenant :
- un support de LED (10) pour être agencé sur ou sous un sujet (100),
- un ensemble (12 ; 24, 26 ; 36) de LED (13 ; 23, 25 ; 33, 35), dans lequel au moins un premier sous-ensemble (12 ; 24 ; 34) de LED (13 ; 23 ; 33) dudit ensemble est agencé dans ou au niveau du support de LED (10),
- une unité de commande (15) pour commander une ou plusieurs LED (13 ; 23 ; 33) dudit premier sous-ensemble (12 ; 24 ; 34) afin de commuter entre un mode émission, dans lequel une LED émet un rayonnement électromagnétique dans la direction du sujet, et un mode détection, dans lequel une LED génère un signal de détection en fonction du rayonnement électromagnétique incident sur la LED,
- une unité de détermination de position (16) pour déterminer la position d'un sujet par rapport au support de LED sur la base des signaux de détection d'une ou de plusieurs LED (13 ; 23 ; 33) dudit premier sous-ensemble (12 ; 24 ; 34) dans le mode détection, et
- une unité de détermination de signes vitaux (17) pour déterminer un ou plusieurs signes vitaux du sujet à partir d'un ou de plusieurs signaux de détection générés par une ou plusieurs LED dudit premier sous-ensemble,
dans lequel l'unité de commande (15) est configurée pour commander une ou plusieurs LED (13 ; 23, 25 ; 33, 35) afin de commuter dans le mode émission sur la base de la position déterminée du sujet de telle sorte que leur rayonnement électromagnétique émis fournit une photothérapie pour le sujet.

2. Système de photothérapie selon la revendication 1,
dans lequel ladite unité de commande (15) est configurée pour commander une LED pour commuter dans le mode émission, si elle est configurée et agencée de telle sorte qu'une quantité substantielle de rayonnement électromagnétique émis par ladite LED touche le sujet.

3. Système de photothérapie selon la revendication 1,
dans lequel ladite unité de détermination de position (16) est configurée pour déterminer si un signal de détection est au-dessus ou au-dessous d'un seuil prédéterminé.

4. Système de photothérapie selon la revendication 3,
dans lequel ladite unité de détermination de position (16) est configurée pour déterminer qu'une LED est sous le sujet si le signal de détection généré par ladite LED dans le mode détection est au-dessous dudit seuil prédéterminé.

5. Système de photothérapie selon la revendication 4,
dans lequel ladite unité de commande (15) est configurée pour commander une LED afin de commuter dans le mode émission uniquement si le signal de détection généré par ladite LED dans le mode détection est au-dessous dudit seuil prédéterminé.

6. Système de photothérapie selon la revendication 3,
dans lequel ladite unité de commande (15) est configurée pour commander une LED afin de se désactiver ou de commuter dans le mode détection si le signal de détection généré par ladite LED dans le mode détection est au-dessus dudit seuil prédéterminé.

7. Système de photothérapie selon la revendication 3,
dans lequel ledit ensemble de LED (13 ; 23, 25 ; 33, 35) est subdivisé en groupes de LED et dans lequel ladite unité de commande (15) est configurée pour commander de façon commune la LED d'un groupe afin de commuter dans le mode émission, si un signal de détection généré par une ou plusieurs LED dudit groupe dans le mode détection est au-dessous dudit seuil prédéterminé.

8. Système de photothérapie selon la revendication 1,
dans lequel ladite unité de commande (15) est configurée pour commander des LED dudit premier sous-ensemble, qui ne sont pas commutées dans le mode émission, afin de commuter dans le mode détection,
dans lequel ladite unité de détermination de position (16) est configurée pour évaluer de façon continue ou régulière des signaux de détection générés par des LED dans le mode détection afin de déterminer si la position du sujet par rapport au support de LED a changé, et
dans lequel ladite unité de commande (15) est configurée pour changer la commande des LED si l'on détermine que la position du sujet par rapport au support de LED a changé.

9. Système de photothérapie selon la revendication 1,
dans lequel toutes les LED (13) dudit ensemble (12) sont agencées dans ou au niveau du support de LED (10).

10. Système de photothérapie selon la revendication 1,
dans lequel une ou plusieurs LED (25 ; 35) d'un second sous-ensemble (26 ; 36) dudit ensemble sont agencées pour être placées au niveau de deux côtés différents ou plus du sujet de telle sorte qu'un rayonnement électromagnétique émis par les LED (25 ; 35) dudit second sous-ensemble touche le sujet par au-dessus, au-dessous et/ou depuis un ou plusieurs côtés.

11. Système de photothérapie selon la revendication 10,
dans lequel ladite unité de commande (15) est configurée pour commander une LED (25 ; 35) dudit second sous-ensemble (26 ; 36) afin de commuter dans le mode émission uniquement si le signal de détection généré par une LED (23 ; 33) du premier sous-ensemble (24 ; 34) dans la même position longitudinale par rapport au sujet est au-dessous dudit seuil prédéterminé et/ou afin d'être désactivée si le signal de détection généré par une LED (23 ; 33) du premier sous-ensemble (24 ; 34) dans la même position longitudinale par rapport au sujet est au-dessus dudit seuil prédéterminé.

12. Système de photothérapie selon la revendication 1,
dans lequel ladite unité de commande (15) est configurée pour commuter seulement les LED (13 ; 23 ; 33) dudit premier sous-ensemble (12 ; 24 ; 34) entre ledit mode émission et ledit mode détection.

13. Système de photothérapie selon la revendication 1,
dans lequel ladite unité de commande (15) est configurée pour commuter au moins une ou plusieurs LED (13 ; 23 ; 33) dudit premier sous-ensemble (12 ; 24 ; 34) dans ledit mode émission sur la base de la position déterminée du sujet.

14. Système de photothérapie selon la revendication 1,
dans lequel ladite unité de détermination de position (16) est configurée pour mesurer une tension de circuit ouvert d'une LED dudit premier sous-ensemble dans le mode détection comme signal de détection.

15. Programme amenant un système de photothérapie comprenant un support de LED (10) pour être agencé sur ou sous un sujet (100) et un ensemble (13 ; 23, 25 ; 33, 35) de LED (12 ; 24, 26 ; 36), dans lequel au moins un premier sous-ensemble (12 ; 24 ; 34) de LED (13 ; 23 ; 33) dudit ensemble est agencé dans ou au niveau du support de LED (10), à réaliser, quand il est exécuté, les étapes suivantes :
- commande d'une ou plusieurs LED (13 ; 23 ; 33) dudit premier sous-ensemble (12 ; 24 ; 34) pour commuter entre un mode émission, dans lequel une LED émet un rayonnement électromagnétique dans la direction du sujet, et un mode détection, dans lequel une LED génère un signal de détection en fonction du rayonnement électromagnétique incident sur la LED,
- détermination de la position d'un sujet par rapport au support de LED sur la base des signaux de détection d'une ou de plusieurs LED (13 ; 23 ; 33) dudit premier sous-ensemble (12 ; 24 ; 34) dans le mode détection,
- détermination d'un ou de plusieurs signes vitaux du sujet à partir d'un ou de plusieurs signaux de détection générés par une ou plusieurs LED dudit premier sous-ensemble, et
- commande d'une ou de plusieurs LED (13 ; 23, 25 ; 33, 35) afin de commuter dans le mode émission sur la base de la position déterminée du sujet de telle sorte que leur rayonnement électromagnétique émis fournit une photothérapie pour le sujet.
